Europäisches Patentamt

European Patent Office

Office européen des brevets

(11) Publication number: **0 310 299**
**A1**

## EUROPEAN PATENT APPLICATION

(21) Application number: **88308801.5**

(22) Date of filing: **22.09.88**

(51) Int. Cl.⁴: **A61K 7/48 , A61K 7/16**

(30) Priority: **28.09.87 US 101625**

(43) Date of publication of application:
**05.04.89 Bulletin 89/14**

(84) Designated Contracting States:
**AT BE CH DE FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Baim, Michael Allen**
**9451 Hunters Creek Drive**
**Cincinnati Ohio 45242(US)**
Inventor: **Koehler, Deborah Adamo**
**5457 Cincinnati-Dayton Road**
**Middletown Ohio 45044(US)**

(74) Representative: **Brooks, Maxim Courtney et al**
**Procter & Gamble (NTC) Limited Whitley**
**Road Longbenton**
**Newcastle-upon-Tyne NE12 9TS(GB)**

(54) **Beta-amino acid ester derivatives of alcoholic actives having extended duration of activity.**

(57) Compositions for application to or consumption by the human body containing $C_3$-$C_6$ beta-amino acid ester derivatives of alcoholic actives, especially alcoholic actives useful as flavorants such as menthol (for example, beta-alanine menthyl ester). These compositions release the alcoholic active over an extended period of time to provide longer lasting activity. Methods for stimulating the cold receptors of the nervous system by administering $C_3$-$C_6$ beta-amino acid ester derivatives of menthol are also taught.

EP 0 310 299 A1

## BETA-AMINO ACID ESTER DERIVATIVES OF ALCOHOLIC ACTIVES HAVING EXTENDED DURATION OF ACTIVITY

### BACKGROUND OF THE INVENTION

The present invention relates to $C_3$-$C_6$ beta-amino acid ester derivatives of alcoholic actives, especially alcoholic actives useful as flavorants (e.g., menthol), in a topically administrable or orally ingestible carrier. These materials release the alcoholic active over an extended period of time to provide longer lasting activity. Furthermore, the present invention relates to methods for stimulating the cold receptors of the nervous system of the human body.

It is frequently desirable to have a material exhibit a longer duration of activity. For example, it is typically desirable to have a flavorant (such as menthol) exhibit a longer lasting cooling effect; or an antibacterial agent exhibit a longer lasting bactericidal activity. Towards this goal, it has been discovered that certain beta-amino acid ester derivatives of alcoholic actives provide this desired extension of duration of activity. This development is particularly useful for extending the flavoring effect of alcoholic flavorants, especially menthol.

For example, menthol is a known alcoholic flavorant active widely used in liquors, confectioneries, perfumes, cigarettes, cough-drops, nasal inhalers, and a wide variety of other compositions for its flavoring and "cooling" properties. Furthermore, menthol is a known topical antipruritic, and has mild local anesthetic and antiseptic activity. Simple esters of menthol, such as menthyl acetate, menthyl borate, menthyl valerate, and menthyl 3-hydroxybutyrate (as described in French Patent Specification 2577922, published August 29, 1986, by Takasago Perfumery KK) are said to have flavoring/cooling properties. Menthyl esters of N-acetyl amino acids, especially N-acetyl glycine menthyl ester, are also known to have cooling effects, as described in U.S. Patent 3,991,178, Humbert et al, issued November 9, 1976. Part of the present invention, however, involves the surprising discovery that certain beta-amino acid menthyl esters are very useful for providing a longer lasting flavoring/cooling sensation in a wide variety of compositions for substantially longer than even closely analogous alpha-amino acid menthyl esters.

It is therefore an object of the present invention to provide compositions containing longer lasting alcoholic actives which are ester derivatives of $C_3$-$C_6$ beta-amino acids. More particularly, an object of the present invention is to provide compositions (especially compositions suitable for contact with the oral cavity) containing longer lasting $C_3$-$C_6$ beta-amino acid ester derivatives of alcoholic flavorant actives (especially menthol), alcoholic perfume actives, or alcoholic antibacterial actives (e.g., thymol). A futher object is to provide compositions for use in the oral cavity containing certain beta-amino acid ester derivatives of alcoholic actives, especially compositions useful as mouth rinses. An object is also to provide methods for stimulating the cold receptors of the nervous system of the human body.

These and other objects of the present invention will become readily apparent from the detailed description which follows.

All percentages and ratios used herein are by weight unless otherwise specified.

### SUMMARY OF THE INVENTION

The present invention relates to compositions for application to or consumption by the human body. These compositions comprise: (a) a $C_3$-$C_6$ beta-amino acid ester derivative of an alcoholic active (preferably a flavorant active, especially menthol); and (b) a topically administrable or orally ingestible carrier.

The present invention further relates to methods for stimulating the cold receptors of the nervous system of the human body. These methods comprise administering to a human a safe and effective amount of a $C_3$-$C_6$ beta-amino acid ester derivative of menthol.

### DETAILED DESCRIPTION OF THE INVENTION

Compositions for Application To or Consumption By The Human Body:

The present invention relates to compositions for application to or consumption by the human body. These compositions essentially comprise: (a) a $C_3$-$C_6$ beta-amino acid ester derivative of an alcoholic active; and (b) a topically administrable or orally ingestible carrier. The essential and optional components for use in the compositions of the present invention, and the amounts utilized, are described in detail hereinafter.

(a) $C_3$-$C_6$ beta-amino acid ester derivatives of alcoholic actives:

The compositions of the present invention essentially comprise an ester derived from an alcoholic active and a beta-amino acid having from 3 to about 6 carbon atoms. The beta-amino acid component of these ester compounds is characterized by having an amine functional group attached to the third carbon atom in a carbon chain terminating in a carboxylic acid group (with the carboxylic acid carbon being counted as the first carbon atom in this chain). For purposes of storage stability in the compositions of the present invention, it is preferred that the amine functional group be predominantly in the protonated (i.e., ammonium salt) form. This is typically achieved by adjusting the pH of the composition as described in more detail hereinafter. It is further preferred that the beta-amino acid component be a three carbon chain (i.e., beta-alanine, which is also known as 3-amin-propionic acid). Most preferred are, therefore, compounds which are esters of beta-alanine predominantly in the ammonium salt form.

The alcoholic active component of the ester compounds useful herein are characterized by having a hydroxy functionality which is capable of forming an ester linkage with a beta-amino acid component as described hereinbefore. Preferred alcoholic active components are those components for which the free alcohol is a known flavorant (referred to herein as "alcoholic flavorant actives"), those components for which the free alcohol is a known perfume (referred to herein as "alcoholic perfume actives"), and those components for which the free alcohol has antibacterial activity (referred to herein as "alcoholic antibacterial actives"). Thus, the $C_3$-$C_6$ beta-amino acid ester derivatives of alcoholic actives are preferably derived from alcoholic actives selected from the group consisting of alcoholic flavorant actives, alcoholic perfume actives, and alcoholic antibacterial actives. It is to be noted that the terms "derivatives of" and "derived from", as used herein, do not mean that the ester compounds are limited to those esters synthesized from the free alcohol, but rather means that the ester compounds may be viewed as having been formed from the free alcohol and the beta-amino acid (whether or not the synthesis of the ester compound involved the use of the free alcohol and/or the beta-amino acid). Examples of alcoholic actives from which the ester compounds useful herein may be derived from include menthol, eugenol, thymol, linalool, beta phenylethyl alcohol, 1-octanol, and 1-nonanol. The most prefered alcoholic flavorant active is menthol, which is also known to produce a cold (or cooling) sensation by stimulating the nervous system of the human body.

Examples of $C_3$-$C_6$ beta-amino acid ester derivatives of alcoholic actives include beta-alanine menthyl ester, 3-aminobutanoic acid menthyl ester, 3-amino-pentanoic acid menthyl ester, beta-alanine eugenyl ester, beta-alanine thymyl ester, and beta-alanine linalool ester. Preferred are beta-alanine thymyl ester and, especially, beta-alanine menthyl ester.

While not intended to be limited by theory, it is believed that the ester compounds utilized in the compositions of the present invention exhibit longer duration of activity for the following reasons. It appears that the ester compounds have an affinity for human tissue, especially the tissue of the oral cavity. This results in the ester compounds being retained by the tissue (e.g., in the oral cavity) for a longer duration of time than the free alcoholic active. While retained by the tissue, the ester slowly hydrolyzes (spontaneously and/or by enzymatic catalysis and/or by other means) to the free alcoholic active and the beta-amino acid. The result is that, for example for a mouthwash, the flavoring/cooling effect of menthol is observed with beta-alanine menthyl ester for substantially longer than rinsing with menthol and, surprisingly, for substantially longer than rinsing with D- or L-alanine menthyl ester (which is an alpha-amino acid).

Synthesis of the $C_3$-$C_6$ beta-amino acid ester derivatives of alcoholic actives is readily achieved by one skilled in the art. In fact, the preferred beta-alanine menthyl ester is a known compound which has been prepared as an intermediate in the synthesis of the menthyl ester of pantothenic acid (see U.S. Patent 2,979,525, Felder et al, issued April 11, 1961; and Felder and Pitre, "Menthyl Esters of Pantothenic Acid", Chimia, 13, pages 168-169 (1959), the disclosures of both being incorporated herein by reference in their entirety). Methods for synthesizing ester compounds for use in the compositions of the present invention are also described in detail in the examples hereinafter.

Preferably, the $C_3$-$C_6$ beta-amino acid ester derivatives of alcoholic actives comprises from about 0.001% to about 10% of the compositions of the present invention, more preferably from about 0.01% to about 2%, and most preferably from about 0.05% to about 1%. The topically administrable or orally ingestible carriers, as described hereinafter, therefore preferably comprise from about 90% to about 99.999%, more preferably from about 98% to about 99.99%, and most preferably from about 99% to about 99.95%, by weight of the present compositions.

(b) Topically administrable or orally ingestible carriers:

The $C_3$-$C_6$ beta-amino acid ester derivatives of alcoholic actives used in this invention find utility in a wide variety of consumer products for consumption by or application to the human body. Broadly speaking, these products can be divided into ingestibles and topicals, both terms being taken in their broadest possible sense. Thus, ingestible is to be taken as including not only foodstuffs and beverages taken into the mouth and swallowed, but also other orally ingested products taken for reasons other than their nutritional value, e.g., indigestion tablets, antacid preparations, laxatives, etc. Ingestible is also to be taken to include edible compositions taken by mouth, but not necessarily swallowed, e.g., chewing gum. Topical is to be taken as including not only compositions such as perfumes, powders and other toiletries, lotions, liniments, oils and ointments, applied to the external surfaces of the human body, whether for medical or other reasons, but also compositions applied to, or which, in normal usage, come in contact with, internal mucous membranes of the body, such as those of the nose (e.g., nasal sprays), mouth, or throat, whether by direct or indirect application, especially mouthwash and gargle compositions. Topical products, in this context, also include toilet articles such as cleansing tissues and toothpicks. Compositions according to the present invention suitable for contact with the oral tissue (whether or not ingested) may utilize ester compounds capable of masking or eliminating mouth malodor and/or sweetening the breath.

In formulating the compositions of this invention, the $C_3$-$C_6$ beta-amino acid ester derivative of an alcoholic active will be incorporated into a topically administrable or orally ingestible carrier by means of which the ester compound may be applied to or ingested by the person. The phrase "topically administrable or orally ingestible carrier", as used herein, means one or more compatible solid or liquid filler diluents or encapsulating substances which are suitable for topical administration to or oral ingestion by the human body. The carrier may itself be completely inert or it may, and usually will, contain active ingredients. A wide variety of carriers will be suitable, depending upon the particular composition involved, with such carriers giving the compositions the form of solids, liquids, emulsions, foams, gels, etc. Typical carriers for the ester compounds include aqueous or alcoholic solutions, oils and fats such as hydrocarbon oils, fatty acid esters, long chain alcohols and silicon oils; finely divided solids such as starch or talc; cellulosic materials such as paper tissue; low-boiling hydrocarbons and halohydrocarbons used as aerosol propellants; gums and natural or snythetic resins. Furthermore, to help prevent or retard hydrolysis of the ester compound during storage, it is preferred that the compositions have a pH of less than about 8, more preferably within a pH range of from about 3 to about 7, and, especially for mouthwashes, most preferably within from about 5 to about 6.

Typically these carriers will contain at least one or more of the following optional components: flavorants (e.g., menthol), colorants, perfuming agents, surface active agents, and/or antiseptic agents, such as are usually employed in topical and ingestible compositions. A more detailed discussion of carriers suitable for particular product compositions according to this invention follows.

A major area of utility of the $C_3$-$C_6$ beta-amino acid ester derivatives of alcoholic actives will be in the field of toilet preparations broadly classed as personal care products. These may be defined as manufactured products applied to the person for the purposes of grooming or hygiene, or for cosmetic purposes, including make up and perfumery. Particular personal care product compositions include, for example, mouthwashes, dentifrices, deodorants, shampoos, etc. They are discussed hereinafter by way of example and are illustrated hereinafter in the specific examples.

One class of personal care products into which the compounds of this invention may be incorporated is represented by lotions for topical application, e.g., aftershave lotions, toilet water, etc., where the ester compound will be used in alcoholic or aqueous alcoholic solution, with such solutions usually also containing a perfume or mild antiseptic or both.

Another class of personal care product is represented by soap and soap-based compositions wherein the ester compounds will be used in combination with an oil or fat, or a natural or synthetic surfactant, e.g., a fatty acid salt or a laurylsulphate salt. These compositions usually also contain an essential oil or perfume. The range of soap compositions will include soaps of all kinds, e.g., toilet soaps, shaving soaps, shaving foams, etc.

A further class of personal care products into which the ester compounds may be incorporated is represented by cosmetic creams, emollients and lotions. Such creams, emollients and lotions usually comprise an oil-in-water emulsion as a base and optionally contain a range of other ingredients such as wax, preservative, perfume, antiseptics, astringents, pigments, etc. Also included within this class are lipstick compositions, such compositions usually comprising an oil and wax base into which the ester compound can be incorporated along with other ingredients, e.g., pigments.

Personal care products for oral hygiene into which the ester compounds, especially those derived from alcoholic flavorant actives (preferably menthol ester derivatives), can be incorporated include mouthwash, gargle and dentifrice compositions. The first two are especially preferred and may be considered together and will usually comprise an aqueous, alcoholic or aqueous-alcoholic solution of one or more antiseptic agents (e.g., cetyl pyridinium chloride; domiphen bromide; tetradecy pyridinium chloride), often colored or flavored for palatability, to which the ester compound (especially beta-alanine menthyl ester) is added. Dentifrice compositions may be of the solid block, powder, paste or liquid type, and will usually comprise a finely divided abrasive or polishing material, e.g., precipitated chalk, silica, magnesium silicate, aluminum hydroxide or other similar materials well known in the art, and a detergent, fluoride source, and/or foaming agent. Optional ingredients which may also be included are flavoring agents and colorants, antiseptics, lubricants, thickeners, emulsifiers or plasticizers.

The ester compounds, especially those derived from alcoholic flavorant actives, may be incorporated into a wide range of edible and potable compositions comprising an edible or potable base and usually one or more flavoring or coloring agents. The particular effect of the esters derived from menthol (e.g., beta-alanine menthyl ester) is to create a cool or fresh sensation in the mouth, and in some cases, even in the stomach. Therefore, the compounds find particular utility in sugar-based confections such as chocolate, boiled sweets, mints and candy, ice cream, jellies, and chewing gums. The formulation of such confections will be traditional techniques and according to conventional recipes. The esters derived from alcoholic flavorant actives will be added to the recipe at a convenient point and in amounts sufficient to produce the desired flavor in the final product. The amount will vary depending upon the particular ester compound, the strength of other flavourants in the recipe, and, in the case of menthyl ester compounds, the degree of cooling effect desired. Similar considerations apply to the formulation of beverages. Generally speaking, the ester compounds used as flavorants in beverages will find most utility in soft drinks, e.g., fruit squashes, lemonade, cola, etc., but may also be used in alcoholic beverages.

Because of their extended duration of activity on the skin, on the mucous membranes of the mouth, throat and nose, and on the gastrointestinal tract, the ester compounds may be used in a variety of oral medicines, nasal and throat sprays, and topical compositions. Generally speaking, these medical preparations, whether topical or ingestible, proprietary or ethical, will contain a pharmaceutically-acceptable carrier, either liquid or solid, which typically comprises one or more pharmaceutically active ingredients. Menthyl ester compounds, especially beta-alanine menthyl ester, are preferred for such compositions in light of their ability to stimulate the cold receptors of the nervous system of the human body (as described hereinafter).

Finally, menthyl ester compounds as described hereinbefore may be utilized in tobacco and tobacco-containing product compositions according to the present invention to create a cooling sensation in the mouth and/or nasal passages. Tobacco and tobacco-containing compositions which may be modified to form compositions according to the present invention (to contain $C_3$-$C_6$ beta-amino acid ester derivatives of menthol) are readily formulated by one skilled in the art (see, for example, U.S. Patent 4,044,120, Rowsell et al, issued August 23, 1977, incorporated herein by reference in its entirety).

Methods for Stimulating Cold Receptors:

The present invention further relates to methods for stimulating the cold receptors of the nervous system of the human body. These methods comprise administering to a human a safe and effective amount of a $C_3$-$C_6$ beta-amino acid ester derivative of menthol, especially beta-alanine menthyl ester.

The menthyl ester is administered topically or orally, as described hereinbefore for the use of the compositions of the present invention. The preferred methods are those whereby the menthyl ester is administered such that it contacts the tissue of the oral cavity to produce a cooling sensation in the mouth, e.g., by rinsing or gargling. The amount of menthyl ester to be administered is generally based on the concentration of the menthyl ester in the carrier. This concentration typically is in the range of from about 0.001% to about 10%, and preferably from about 0.01% to about 2%, by weight of the composition being administered. If administered by way of the preferred mouthwash solutions, the preferred concentration is typically within the range of from about 0.05% to about 1%, and preferably from about 0.1% to about 0.2%, by weight of the rinse solution.

The following examples further describe and demonstrate embodiments within the scope of the present invention. The examples are given solely for the purpose of illustration and are not to be construed as limitations of the present invention as many variations thereof are possible without departing from the spirit and scope.

## EXAMPLE I

### Synthesis of Beta-Alanine, (-)-Menthyl Ester (Free Base)

The reaction is carried out in two duplicate runs. A stirred suspension of beta-alanine (750 g, 8.41 mol; Aldrich Chemical Co., Inc.), (1R,2S,5R)- (-)-menthol (2050 g, 13.12 mol; Aldrich Chemical Co., Inc.), p-toluenesulfonic acid monohydrate (1683 g, 8.847 mol; Aldrich Chemical Co., Inc.) and benzene (8 L) is heated at reflux in a 22 L flask for 7 days while water is collected in a Dean-Stark trap. By the sixth day a clear solution has resulted. The hot solution is allowed to cool overnight, after which time the product partially crystallizes. The mixture is diluted with hexanes (4 L) and stirred, filtered, and the residue washed with hexanes (2 x 2 L). The combined solid from both runs is stirred with hexanes (15 L) and then filtered. The residue is washed with hexanes (2 x 5 L) and then allowed to air dry. Further drying is carried out in vacuo to afford the p-toluenesulfonate salt of beta-alanine, (-)-menthyl ester which is suitable for subsequent conversion.

A solution of sodium hydroxide (331 g, 8.29 mol) in 2.5 L of ice water is added to a mixture of beta-alanine, (-)-menthyl ester p-toluenesulfonate (2588 g, 6.477 mol), hexanes (5 L) and 3 L of ice water. The mixture is stirred for 10 min. The aqueous layer is then separated and extracted with hexanes (2 x 2 L). The combined cloudy hexane extracts are washed with ice water (2 x 2 L). The still cloudy solution is concentrated in vacuo to about 5 L. The now clear solution is treated with anhydrous sodium sulfate, filtered, and the residue of drying agent is washed with hexanes (3 x 200mL). The filtrate is divided into two nearly equal portions and evaporated in vacuo in 3-L flasks. Each portion of residue is evaporated in vacuo with hexanes (2 x 800 mL), and then is stored overnight at -10°C. The material, which by now has formed a small amount of white powder, is diluted with hexanes (400 mL) and filtered through a bed of Celite filter aid. The clear filtrate is concentrated in vacuo to a cloudy liquid. The material, stored under argon, is distilled in portions with a Kugelrohr apparatus. A small forerun (bp 85-95°C/0.1 torr) is collected and discarded. The Kugel ohr receiver is cleaned, and the distillation is continued to afford colorless material (bp 90-105°C/0.1 torr). The distilled material from the various distillations is combined; and further conversion of 3679 g of beta-alanine, (-)-menthyl ester p-toluenesulfonate by the above procedure gives additional beta-alanine, (-)-menthyl ester suitable for use in the compositions of the present inventions. In order to preserve this free base form, the products are stored under an argon atmosphere to protect it from reacting with $CO_2$ in the atmosphere to form the white solid ammonium carbonate salt (which may also be used to formulate compositions according to the present invention).

## EXAMPLE II

### Mouthwash Solution

A mouthwash composition according to the present invention is prepared having the following components:

| Component | Weight % |
|---|---|
| Alcohol | 12.000 |
| Flavor | 0.120[2] |
| Tween 80 | 0.300 |
| DRO Water | 71.515 |
| Benzoic Acid | 0.055 |
| Monosodium Phosphate | 0.700 |
| Beta-Alanine, Menthyl Ester[1] | 0.150 |
| Cetyl Pyridinium Chloride | 0.045 |
| Domiphen Bromide | 0.005 |
| Sorbitol | 15.000 |
| Saccharin | 0.070 |
| FD&C Blue #1 | 0.020 |
| Yellow #5 | 0.020 |

[1] Free base which is prepared as described in Example I.
[2] Flavor system = rectified peppermint oil (supplied, for example, by Wm. Leman, Inc.; Bremen, Indiana).

This mouthwash composition is prepared by mixing the components in the order listed, waiting until the solution clears before the next component is added. The final composition has pH within the range of about 5 - 6. Use of this composition by rinsing the mouth for a few seconds provides a long lasting cooling sensation.

## EXAMPLE III

### Dentifrice

A dentifrice composition according to the present invention is prepared having the following components:

| Component | Weight % |
|---|---|
| Sorbitol | 50.925 |
| Water | 12.500 |
| Saccharin | 0.250 |
| Titanium Dioxide | 0.525 |
| FD&C #1 Blue | 0.050 |
| Silica | 20.000 |
| Glycerin | 10.000 |
| Carboxymethyl Cellulose | 0.900 |
| Carrageenan | 0.350 |
| Sodium Alkyl Sulfate (27% solution) | 4.000 |
| Beta-alanine Thymyl Ester[1] | 0.500 |

[1] Free base which is prepared by a synthesis procedure analogous to Example I.

These components are blended, and the pH of the composition is adjusted to approximately pH = 7. Use of this dentifrice composition provides long lasting antiseptic effect in the mouth.

## EXAMPLE IV

### After-Shave Lotion

An after-shave lotion is prepared according to the following recipe by dissolution of the ingredients in the liquid and cooling and filtering:

| Component | Weight % |
|---|---|
| Denatured ethanol | 75 |
| Diethylphthalate | 1.0 |
| Propylene Glycol | 1.0 |
| Lactic Acid | 1.0 |
| Perfume | 3.0 |
| Water | to 100 |

Into the base lotion is added 0.5% by weight of beta-alanine menthyl ester. When the final solution is applied to the face, a clearly noticeable cooling effect is apparent after a short interval of time.

## EXAMPLE V

### Antiseptic Ointment

An ointment is prepared according to the following formulation:

| Component | Weight % |
|---|---|
| Cetyltrimethyl Ammonium Bromide | 4.0 |
| Cetyl Alcohol | 6.0 |
| Stearyl Alcohol | 6.0 |
| White Paraffin | 14.0 |
| Mineral Oil | 21.0 |
| Water | to 100 |

The ingredients are mixed, warmed to 40°C and emulsified in a high speed blender. Added to the mixture during blending is 1.5% of beta-alanine menthyl ester, and the pH is adjusted to approximately pH = 6. The final ointment, when applied to the skin, gives rise to a cooling effect.

## EXAMPLE VI

### Soft Drink

A soft drink concentrate is prepared from the following recipe:

EP 0 310 299 A1

| Component | Weight % |
|---|---|
| Pure Orange Juice | 60 |
| Sucrose | 10 |
| Saccharin | 0.2 |
| Orange Flavoring | 0.1 |
| Citric Acid | 0.2 |
| Sulphur Dioxide | trace amount |
| Water | to 100 |

To the concentrate is added 0.10% of beta-alanine menthyl ester. The concentrate is diluted with water and tasted. An orange flavor having a pleasantly cool after-effect is obtained.

EXAMPLE VII

Hair Shampoo

Sodium lauryl ether sulphate, 10 g., is dispersed in 90 g. water in a high speed mill. To the dispersion is added 2%, by weight, of beta-alanine menthyl ester, and the pH adjusted to approximately pH = 6. When the hair is washed using the shampoo, a fresh, cool sensation is noticed on the scalp.

Claims

1. Compositions for application to or consumption by the human body comprising:
   (a) a $C_3$-$C_6$ beta-amino acid ester derivative of an alcoholic active; and
   (b) a topically administrable or orally ingestible carrier.

2. Compositions according to Claim 1 wherein the $C_3$-$C_6$ beta-amino acid ester derivative of an alcoholic active is derived from alcoholic actives selected from the group consisting of alcoholic flavorant actives, alcoholic perfume actives, or alcoholic antibacterial actives.

3. Compositions according to Claims 1 or 2 wherein the $C_3$-$C_6$ beta-amino acid ester derivative of an alcoholic active is derives from alcoholic actives selected from the group consisting of menthol and thymol.

4. Compositions according to any of Claims 1-3 comprising:
   (a) from 0.001% to 10% of a beta-alanine ester derivative of an alcoholic active; and
   (b) from 90% to 99.999% of a topically administrable or orally ingestible carrier.

5. Compositions according to any of Claims 1-4 wherein the beta-alanine ester derivative of an alcoholic active is selected from the group consisting of beta-alanine menthyl ester or beta-alanine thymyl ester.

6. Compositions for application to or consumption by the human body comprising:
   (a) from 0.001% to 10% of beta-alanine menthyl ester; and
   (b) from 90% to 99.999% of a topically administrable or orally ingestible carrier.

7. Compositions according to Claim 6 wherein the topically administrable or orally ingestible carrier comprises one or more components selected from the group consisting of flavorants, colorants, perfuming agents, surface active agents, or antiseptic agents.

8. Compositions according to Claims 6 or 7 wherein the topically administrable or orally ingestible carrier comprises a component selected from the group consisting of water, alcohol, and mixtures thereof.

9. Compositions suitable for use as a dentifrice according to any of Claims 6-8 comprising:
   (a) from 0.01% to 2% of beta-alanine menthyl ester; and
   (b) from 98% to 99.99% of a topically administrable or orally ingestible carrier comprising finely divided abrasive or polishing material.

10. Compositions suitable for use as a mouthwash comprising:

9

(a) from 0.05% to 1% of beta-alanine menthyl ester: and

(b) from 99% to 99.95% of a topically administrable or orally ingestible carrier comprising a liquid selected from the group consisting of water, alcohol, and mixtures thereof.

11. Compositions according to Claim 10 wherein the topically administrable or orally ingestible carrier further comprises one or more components selected from the group consisting of flavorants, colorants, or antiseptic agents.

12. Compositions according to Claims 10 or 11 comprising menthol.

13. The compound beta-alanine thymyl ester, and ammonium salts thereof.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y,D | FR-A-2 236 844 (UNILEVER)<br>* Whole document * & US-A-3 991 178<br>--- | 1-12 | A 61 K 7/48<br>A 61 K 7/16 |
| Y | CHEMICAL ABSTRACTS, vol. 89, no. 16, October 1978, page 389, no. 135685k, Columbus, Ohio, US; & JP-A-78 06 215 (NIPPON CHEMICAL CO., LTD) 06-03-1978<br>* Abstract *<br>--- | 1-12 | |
| X | DE-A-2 025 629 (O. MANZKE)<br>* Page 5, point 4 * | 1 | |
| Y | ---| 2-12 | |
| Y | THE MERCK INDEX, ENCYLOPEDIA OF CHEMICALS, DRUGS AND BIOLOGICALS, 10th edition, 1983, page 957, no. 6518, Merck & Co., Rahway, US; M. WINDHOLZ et al.: "Nonoxynol"<br>* Whole article *<br>--- | 1-12 | |
| A | DE-A-2 022 369 (J.A. SEBALD GmbH)<br>* Page 2, lines 4-16; examples *<br>--- | 1-12 | TECHNICAL FIELDS SEARCHED (Int. Cl.4) |
| A | US-A-3 793 446 (A. MOELLER et al.)<br>* Claims; column 3, lines 35-50 *<br>--- | 1-12 | A 61 K 7/00<br>A 23 L 1/00 |
| A | DE-A-3 211 913 (M. MARKS)<br>* Claim 1 *<br>--- | 1-12 | |
| A | GB-A-1 436 614 (K. THOMAE GmbH)<br>* Whole document *<br>---        -/- | 1-12 | |

The present search report has been drawn up for all claims

| Place of search | Date of completion of the search | Examiner |
|---|---|---|
| THE HAGUE | 15-12-1988 | GERLI P.F.M. |

EPO FORM 1503 03.82 (P0401)

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | CHEMICAL ABSTRACTS, vol. 77, no. 19, 6th November 1972, page 441, no. 127049t, Columbus, Ohio, US; M. LIDAKS et al.: "Synthesis of peptides based on beta-(1-pyrimidyl)- and beta-(9-purinyl)-alpha-amino acids", & KHIM. GETEROTSIKL. SOEDIN. 1972, (5), 705-7 | 13 | |

TECHNICAL FIELDS
SEARCHED (Int. Cl.4)

The present search report has been drawn up for all claims

| Place of search | Date of completion of the search | Examiner |
|---|---|---|
| THE HAGUE | 15-12-1988 | GERLI P.F.M. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................................................

& : member of the same patent family, corresponding
    document

EPO FORM 1503 03.82 (P0401)